# EUROPEAN PATENT SPECIFICATION

(11) **EP 1 214 951 B1**
(45) Date of publication and mention of the grant of the patent: **15.09.2004**
(21) Application number: 01127485.9
(22) Date of filing: 28.11.2001
(51) Int. Cl.: A61L 27/16, A61L 31/10

(54) **Expanded polytetrafluoroethylene product for medical applications**
Expandiertes Polytetrafluoroethylen für medizinische Anwendungen
Polytétrafluoroéthylène expansé pour applications médicales

(30) Priority: 13.12.2000 DE 10061936
(43) Date of publication of application: 19.06.2002
(73) Proprietor: Kramer, Valentin, 83620 Feldkirchen-Westerham (DE); Ruefer, Bruce G., Bozeman, MT 59718 (US)
(72) Inventor: Kramer, Valentin, 83620 Feldkirchen-Westerham (DE); Ruefer, Bruce G., Bozeman, MT 59718 (US)
(74) Representative: von Hellfeld, Axel, Dr. Dipl.-Phys.

(56) References cited:
- EP-A- 0 402 901
- US-A- 4 822 361
- US-A- 5 708 044
- US-A- 5 843 171
- US-A- 5 980 799

## Description

### FIELD OF THE INVENTION

The present invention relates generally to composite articles formed from expanded polytetrafluoroethylene ("ePTFE") materials, and particularly to a composite article made up of a plurality of polytetrafluoroethylene ("PTFE") components having differing expansion characteristics such that differing ePTFE structures are exhibited.

### BACKGROUND OF THE INVENTION

DE 690 03 879 describes a porous, at least uni-axially expanded PTFE material comprising a mixture of a PTFE having a high molecular weight of 2.000.000 or more and a PTFE having a low molecular weight of 1.000.000 or less. The size of the pores of the PTFE-material can be varied by changing the mixing ratio for the PTFE with high molecular weight and the PTFE with low molecular weight. The PTFE-material can exhibit different shapes, for example a foil, sheet or cube. Further, the PTFE-material can be used in different fields, for example as membrane filter exhibiting a low pressure loss as diaphragma, as smearing glide means and as bonding or sticking means, respectively.

Many similar designs of ePTFE tubes serving as vascular grafts ("grafts") can be found in the market place. These designs range from a fairly simple uniaxially expanded ePTFE graft made into various bore sizes (W.L. Gore & Associates, Flagstaff, Arizona) and lengths to more complex design of uniaxially expanded ePTFE tube reinforced with a ring complex made of fluorinated ethylene propylene ("FEP") or ePTFE film (W.L. Gore & Associates, Flagstaff, Arizona). In addition, double wall ePTFE grafts constructed as a "tube within a tube" can be found in the patent literature (US Pat. 5,935,667). Most of these grafts are designed to exhibit a uniform structure of fibrils and nodes containing about 30 micron pores.

This pore size is believed to be advantageous for blood contact, control bleeding, and make the graft adequately strong.

US-5,843,171 discloses vascular grafts made of porous expanded PTFE having areas of differing microstructure, i.e. different areas have different mean fibril lengths. Further, it is disclosed that a mean value of around 4 µm for the small fibril areas and a mean value of around 17 µm for the large fibril areas are employed. These vascular grafts are obtained by a process using a single PTFE resin.

US-5,980,799 relates to a method of forming porous e-PTFE articles of varying pore size distribution. By varying degree and nature of lubricant and the stretching conditions of a single PTFE resin, tubular objects are obtained. These tubular objects have large pores on the internal and external walls and include a barrier region of smaller pore sizes between both surfaces. Such structures are used as vascular implants.

US-4,822,361 relates to a tubular e-PTFE prosthesis having a greater average fibril length on the outer surface than on the inner surface. Further, it is disclosed to extrude a tubular e-PTFE structure from a single resin, wherein the resulting structure has a mean pore size of 2 µm at the interior surface and 150 µm at the exterior surface.

While the ePTFE vascular grafts are reported to be functional for their intended use, significant and novel design improvements are needed to address the known inadequacies of their designs that relate to optimum blood contact requirements, strength requirements, and pore size distribution. The invention disclosed herein and defined in claims 1 and 10 accomplishes this goal.

### BRIEF DISCUSSION OF THE INVENTION

The invention provides an article according to claim 1 and a method according to claim 9. The article contains a novel fibril and node structure that exhibits a pore size distribution of two or more distinct pore sizes. The pore size distribution of small pores inter-spaced with larger pores to create a mosaic pore structure is advantageous as a blood-contacting surface and renders the invention a very useful and advantageous vascular graft, cardio vascular patch, cardio vascular suture, stent cover, and comparable medical devices and means.

The preferred invention disclosed herein consists of an ePTFE tube comprising two or more PTFE (polytetrafluoroethylene) resins that are blended, stretched, and sintered or locked into a novel fibril and node matrix. The tube is constructed to exhibit pores within the matrix of fibrils and nodes that exhibit two or more distinct size-distributions of pores. The preferred invention may be reinforced with an outer wrapping of a Fluorinated Ethylene Propylene (FEP) filament configured into a double helix structure. The advantages of the preferred invention will come forth as the details are disclosed herein.

According to the invention, there are provided two distinct groups of pores in the ePTFE (expanded polytetrafluoroethylene). A first group consists of pores the sizes of which are in the range of 2 micron to 15 micron, preferably in the range from 3 micron to 8 micron, most preferably in the range from 4 micron to 6 micron, in particular around 5 micron. A second group consists of pores having sizes which are in, the range from 20 micron to 50 micron, in particular in the range from 25 to 40 micron, most preferably the range from 25 to 35 micron, in particular around 30 micron.

The afore-mentioned at least two distinct groups of pores are preferably randomly distributed in the ePTFE tube material. The smaller pores are found within the larger pores, according to a statistical (random) distribution of pores.

As to the number of pores of smaller size as compared to the number of pores of larger size, the afore-mentioned preferred embodiment comprising at least two distinct groups of pores, the invention discloses a ratio of number of pores per volume unit of expanded PTFE of the first group and the number of pores per volume unit of expanded PTFE of the second group, said ratio being selected in the range from 0,2 to 5, preferably 0,4 to 3, most preferably in the range of 0,6 to 2, in particular the ratio can have a value of 1 ± 0,2.

The afore-mentioned embodiment of the invention comprising two distinct groups has turned out to be most efficient with regard to the above stated problem.

The preferred invention may be constructed in a variety of shapes and sizes, such as a tube with or without the reinforcing wrapping, or a sheet, or a reinforced sheet.

### BRIEF DESCRIPTION OF THE DRAWINGS

Figure 1 is a two-dimensional drawing showing the ePTFE tube with outer reinforcing wrapping of the preferred invention.
Figure 2 a two-dimensional drawing showing the novel bi-pore mosaic structure of the preferred invention.
Figure 3 is a 500X scanning electron micrograph (SEM) of the novel bi-pore mosaic ePTFE structure of the preferred invention.
Figure 4 is a 100X scanning electron micrograph (SEM) of the novel bi-pore mosaic ePTFE structure of the preferred invention.

### DETAILED DESCRIPTION OF THE DRAWINGS

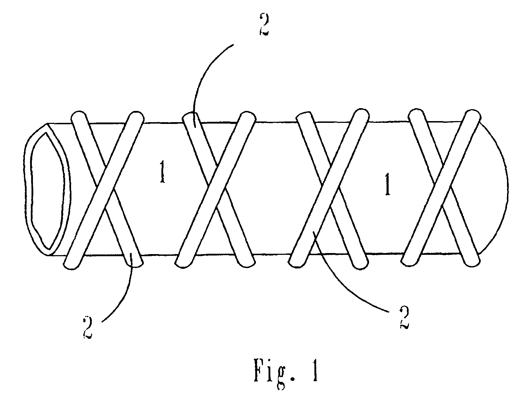

- Fig. 1: depicts a two-dimensional overview drawing of the preferred invention showing the novel ePTFE tube 1 with a FEP filament wrap 2 reinforcing the tube.

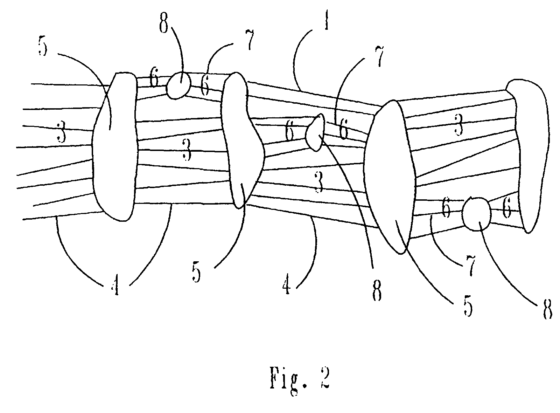

- Fig. 2: shows a close up two-dimensional drawing of the pre ferred invention showing two distinct pore size distributions. The larger pores 3 are shown as a distribution within the structure and contain long fibril structures 4 connected between large solid PTFE node structures 5. The small pores 6 are shown as a distribution within the larger pores 3 and are shown containing short fibril structures 7 connected between small solid PTFE node structures 8 and other small solid PTFE node structures or, as shown in fig ure 2, large solid PTFE node structures 5. The smaller pore size distributions are found within the larger pore size distribution in a random manner forming a bi-pore mosaic overall structure. As is shown in Fig. 2, a cross-section through the material displays first areas of the smaller pore size distri bution and second areas distinct from the smaller pore size areas, the second areas being larger, according to the larger pore size distribution. The ratio of the first and second areas (each area measured in µm²) is preferably selected from the range of 1:5 to 1:1.

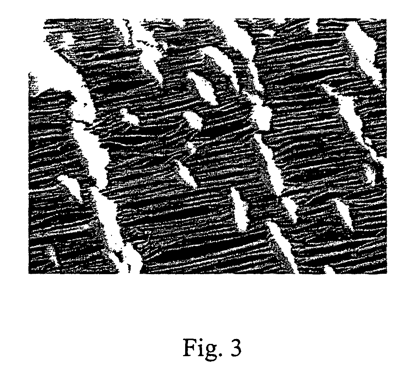

- Fig. 3: is a scanning electron micrograph (SEM) of the novel structure of the preferred invention at 500X. The SEM shows the two distinct pore size distributions forming a mosaic pore structure advantageous for the invention.

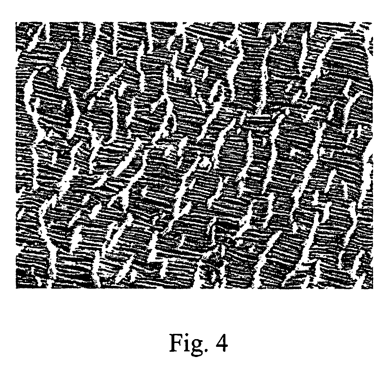

- Fig. 4: is a scanning electron micrograph (SEM) of the novel structure of the preferred invention at 100 X. The SEM depicts more closely the two distinct pore size distributions forming a mosaic pore structure advan tageous for the invention.

The preferred invention is made in the following manner: Two PTFE resins are chosen based on the following properties. (1) A resin that expands to exhibit a relatively small pore size distribution of about 5 microns. (2) A resin that expands to exhibit a relatively large pore size distribution of about 30 microns. The resins are mixed homogenously to about a 1: 1 ratio and then blended with a lubricant. The resultant paste is formed into a billet with medium pressure in a pelletizer apparatus. The billet is extruded into a tube. The resultant extruded PTFE tube is then expanded with heat to make the ePTFE structure. The resultant ePTFE tube is reinforced with an outer FEP filament wrap configured into a double helix structure. The reinforced tube is heat treated to fuse the FEP filament to the outer portion of the ePTFE tube.

In the afore-mentioned general description of the preferred embodiment, the ratio of 1:1 of the two resins can be varied in certain ranges, preferably the weight ratio can be varied in the range from 0,5:1 to 2:1, most preferably in the range from 0,75:1 to 1,25:1. Furthermore, the resins can be selected to produce other pore sizes, the most preferred ranges being stated above.

The resulting ePTFE tube exhibits the following properties: The inner wall and surface structure of the ePTFE tube exhibits a mosaic bi-pore structure of fibrils and nodes. The novel bi-pore mosaic ePTFE tube is a structure exhibiting two distinct pore size distributions found to be randomly inter-spaced one within the other.

### EXAMPLE I:

Two polytetrafluoroethylene (PTFE) resins are selected according to their expansion characteristics as follows:
(1) A high molecular weight grade of resin (about 3 million Daltons) is selected to select for small pore sizes of about 5 microns.
(2) A low molecular weight grade of resin (about 1 million Daltons) is selected to select for large pore sizes of about 30 microns. The resins are weighed to make a ratio of about 50 / 50 by weight and are simultaneously blended with a lubricant until thoroughly mixed and coated with lubricant. The resultant resin paste is then made into a billet per standard practice with a billet making apparatus called a pelletizer. The billet is then warmed to about 35 °C and is inserted into a ram extruder. Forcing the PTFE billet through a die with high-pressure forms a PTFE tube. The tube is then expanded in a linear manner at about the melt point of the PTFE of about 350 °C. The resultant expanded PTFE (ePTFE) tube is then cut to various lengths. The tubes are reinforced with FEP helix wrapping by inserting a precision stainless tube into the ePTFE tube and then wrapping the FEP onto the ePTFE tube. The FEP wrapping is secured to the underlying ePTFE tube by heating the assembly in an oven at or near the melting point of the FEP.

The resulting ePTFE tubes are examined and show the following characteristics:
(1) a fibril and node structure containing two distinct pore size distributions wherein one is found within another; and
(2) A very flexible tube showing excellent resistance to kinking upon bending at 180 degrees.

## Claims

1. An article of expanded PTFE wherein said article is selected from the group consisting of a vascular graft, a cardio vascular patch, a cardio vascular suture, or a stent cover, the article of expanded PTFE exhibiting a fibril and node structure containing two or more distinct pore sizes distributions, one within another, wherein one pore size distribution comprises smaller pore sizes than another pore size distribution and the pores of the smaller pore size distribution are randomly distributed within the pores of the larger pore size distribution, wherein the smaller pore sizes are in the range of 2 to 15 microns and the pores of the larger pore size distribution are in the range of 20 to 50 microns.

2. An article according to claim 2, wherein the smaller pore sizes are in the range of 3 to 8 microns and the pores of the larger pore size distribution are in the range from 25 to 40 microns.

3. An article according to claim 1 or 2, wherein the smaller sizes are in the range from 4 to 6 microns and the pores for the larger pore size distribution are in the range from 25 to 35 microns.

4. An article according to one of the preceding claims, wherein the smaller pore sizes are around 5 microns and the pores for the larger pore size distribution are around 30 microns.

5. An article according to one of the preceding claims, that is configured into a tube.

6. An article according to claim 5, that is configured into a reinforced tube.

7. An article according to one of the claims 1 to 4, that is configured into a sheet.

8. An article according to claim 7, that is configured into a reinforced sheet.

9. A method for producing a vascular graft, cardio vascular patch, cardio vascular suture, or stent cover from expanded PTFE, said method comprising the steps of:
- selecting a first PTFE resin that expands to exhibit a relatively small pore size distribution, wherein the small pore size is in the range from 2 to 15 microns,
- selecting a second PTFE resin that expands to exhibit a relatively large pore size distribution, wherein the large pore size is in the range from 20 to 50 microns,
- mixing the first and second resins and, if any, further resins, homogeneously and blending them with a lubricant, such that, after expanding, the pores of the smaller pore size distribution are randomly distributed within the pores of the larger pore size distribution,
- forming the such obtained blend into a billet,
- extruding the billet into a tube or sheet, and
- expanding the extruded PTFE tube or sheet and heating it.

10. The method according to claim 9, wherein the small pore size is in the range from 3 to 8 microns and the large pore size is in the range from 25 to 40 microns.

11. The method according to claim 9 or 10, wherein the small pore size is in the range from 4 to 6 microns and the large pore size is in the range from 25 to 35 microns.

12. The method according to one of the claims 9 to 11, wherein the small pore size is around 5 microns and the large pore size is around 30 microns.

## Patentansprüche

1. Produkt aus expandiertem PTFE, das aus der Gruppe bestehend aus einer Aderprothese, einem kardiovaskulären Patch, kardiovaskilärem Nahtmaterial oder einer Stentabdeckung gewählt wurde, wobei das Produkt aus expandiertem PTFE eine Faser- und Knotenstruktur hat, die zwei oder mehr unterschiedliche Porengrößenverteilungen aufweist, wobei die eine Verteilung in der anderen verschachtelt ist und eine Porengrößenverteilung kleinere Porengrößen aufweist als die andere Porengrößenverteilung und die Poren der kleineren Porengrößenverteilung innerhalb der Poren der größeren Porengrößenverteilung statisch (zufällig) verteilt sind, wobei die kleineren Porengrößen im Bereich von 2 bis 15 Mikron und die Poren der größeren Porengrößenverteilung im Bereich von 20 bis 50 Mikron liegen.

2. Produkt gemäß Anspruch 1, bei dem die kleineren Porengrößen im Bereich von 3 bis 8 Mikron und die Poren der größeren Porengrößenverteilung im Bereich von 25 bis 40 Mikron liegen.

3. Produkt gemäß Anspruch 1 oder 2, bei dem die kleineren Größen im Bereich von 4 bis 6 Mikron und die größere Porengrößenverteilung im Bereich von 25 bis 35 Mikron liegen.

4. Produkt gemäß einem der vorhergehenden Ansprüche, bei dem die kleineren Porengrößen etwa 5 Mikron und die Poren für die größere Porengrößenverteilung bei etwa 30 Mikron betragen.

5. Produkt gemäß einem der vorhergehenden Ansprüche, in Rohrform.

6. Produkt gemäß Anspruch 5, in verstärkter Rohrform.

7. Produkt gemäß einem der Ansprüche 1 bis 4, in Platten- oder Blattform.

8. Produkt gemäß Anspruch 7, bei dem die Platte bzw. das Blatt verstärkt ist.

9. Verfahren zum Herstellen von einer Aderprothese, einem kardiovaskulären Patch, kardiovaskilärem Nahtmaterial oder einer Stentabdeckung aus expandiertem PTFE, wobei das Verfahren folgende Schritte umfasst:
- Auswählen eines ersten PTFE-Materials, das so expandiert, dass sich eine Porengrößenverteilung mit relativ kleinen Poren ergibt, wobei die kleine Porengröße im Bereich von 2 bis 15 Mikron liegt;
- Auswählen eines zweiten PTFE-Materials, das so expandiert, dass sich eine Porengrößenverteilung mit relativ großen Poren ergibt, wobei die große Porengröße im Bereich von 20 bis 50 Mikron liegt;
- homogenes Mischen der ersten und zweiten Materialien und gegebenenfalls weiterer Materialien und Vermischen derselben mit einem Gleitmittel derart, dass nach dem Expandieren die Poren der kleineren Porengrößenverteilung statisch (zufällig) innerhalb der Poren der größeren Porengrößenverteilung verteilt sind;
- Formen der so erhaltenen Mischung in Barren;
- Extrudieren der Barren zu Rohr- oder Platten- bzw. Blattform und
- Expandieren des extrudierten PTFE-Röhrchens oder der Platte bzw. des Blattes und Erhitzen derselben.

10. Verfahren gemäß Anspruch 9, bei dem die kleine Porengröße im Bereich von 3 bis 8 Mikron und die große Porengröße im Bereich von 25 bis 40 Mikron liegt.

11. Verfahren gemäß Anspruch 9 oder 10, bei dem die kleine Porengröße im Bereich von 4 bis 6 Mikron und die große Porengröße im Bereich von 25 bis 35 Mikron liegt.

12. Verfahren gemäß einem der Ansprüche 9 bis 11, bei dem die kleine Porengröße bei etwa 5 Mikron und die große Porengröße bei etwa 30 Mikron liegt.

## Revendications

1. Article en PTFE expansé, ledit article est sélectionné parmi le groupe consistant en une prothèse vasculaire, un patch cardio-vasculaire, une suture cardio-vasculaire ou une endoprothèse couverte, l'article en PTFE expansé présente une structure de fibrilles et de noeuds contenant deux ou plus de deux distributions de tailles de pores distinctes, disposées l'une à l'intérieur de l'autre, dans lesquelles l'une des distributions de pores comprend des pores de plus petite taille que l'autre distribution de pores et les pores de la distribution de pores de plus petite taille sont répartis de manière aléatoire à l'intérieur des pores de la distribution de pores de plus grande taille, dans lesquelles les pores de plus petite taille se situent dans la plage allant de 2 à 15 microns et les pores de la distribution de pores de plus grande taille se situent dans la plage allant de 20 à 50 microns.

2. Article selon la revendication 1, dans lequel les pores de plus petite taille se situent dans la plage allant de 3 à 8 microns et les pores de la distribution de pores de plus grande taille se situent dans la plage allant de 25 à 40 microns.

3. Article selon la revendication 1 ou 2, dans lequel les pores de plus petite taille se situent dans la plage allant de 4 à 6 microns et les pores de la distribution de pores de plus grande taille se situent dans la plage allant de 25 à 35 microns.

4. Article selon l'une des revendications précédentes, dans lequel les pores de plus petite taille se situent autour de 5 microns et les pores de la distribution de pores de plus grande taille se situent autour de 30 microns.

5. Article selon l'une des revendications précédentes, configuré en un tube.

6. Article selon la revendication 5, configuré en un tube renforcé.

7. Article selon l'une des revendications 1 à 4, configuré en une feuille.

8. Article selon la revendication 7, configuré en une feuille renforcée.

9. Procédé de production d'une prothèse vasculaire, d'un patch cardio-vasculaire, d'une suture cardio-vasculaire ou d'une endoprothèse couverte à partir de PTFE expansé, ledit procédé comprenant les étapes consistant à :
- sélectionner une première résine de PTFE expansible qui présente une distribution de pores de taille relativement petite dans laquelle les pores de petite taille se situent dans la plage allant de 2 à 15 microns,
- sélectionner une seconde résine de PTFE expansible qui présente une distribution de pores de taille relativement grande dans laquelle les pores de grande taille se situent dans la plage allant de 20 à 50 microns,
- mélanger les première et seconde résines et, le cas échéant, toute autre résine, de manière homogène et les amalgamer avec un lubrifiant de telle sorte qu'après l'expansion, les pores de la distribution de pores de plus petite taille soient répartis de manière aléatoire à l'intérieur de la distribution de pores de plus grande taille,
- configurer le mélange ainsi obtenu en une billette,
- extruder la billette pour former un tube ou une feuille, et
- expanser le tube ou la feuille de PTFE extrudé et le/la chauffer.

10. Procédé selon la revendication 9, dans lequel les pores de petite taille se situent dans la plage allant de 3 à 8 microns et les pores de grande taille se situent dans la plage allant de 25 à 40 microns.

11. Procédé selon la revendication 9 ou 10, dans lequel les pores de petite taille se situent dans la plage allant de 4 à 6 microns et les pores de grande taille se situent dans la plage allant de 25 à 35 microns.

12. Procédé selon l'une des revendications 9 à 11, dans lequel les pores de petite taille se situent autour de 5 microns et les pores de grande taille se situent autour de 30 microns.
